# EUROPÄISCHE PATENTSCHRIFT

(11) **EP 3 606 399 B1**
(45) Veröffentlichungstag und Bekanntmachung des Hinweises auf die Patenterteilung: **01.05.2024**
(21) Anmeldenummer: 18719763.7
(22) Anmeldetag: 06.04.2018
(51) Int. Cl.: A61B 1/00

(54) **ENDOSKOP MIT ZUSÄTZLICHEM EXTERNEM ARBEITSKANAL**
ENDOSCOPE WITH ADDITIONAL EXTERNAL WORKING CHANNEL
ENDOSCOPE POURVU D'UN CANAL DE TRAVAIL EXTERNE SUPPLÉMENTAIRE

(30) Priorität: 07.04.2017 DE 102017107546
(43) Veröffentlichungstag der Anmeldung: 12.02.2020
(73) Patentinhaber: Ovesco Endoscopy AG, 72076 Tübingen (DE)
(72) Erfinder: HO, Chi-Nghia, 72762 Reutlingen (DE); MEINING, Alexander, 89075 Ulm (DE)
(74) Vertreter: Winter, Brandl - Partnerschaft mbB
(86) Internationale Anmeldenummer: PCT/EP2018/058874
(87) Internationale Veröffentlichungsnummer: WO 2018/185293

(56) Entgegenhaltungen:
- EP-A1- 1 284 120
- WO-A1-2005/039427
- DE-A1- 10 356 018
- DE-A1-102007 008 099
- DE-A1-102009 014 178
- JP-A- H07 155 283
- JP-B2- 4 441 232
- US-A1- 2008 277 853
- US-A1- 2013 310 641
- US-A1- 2016 206 178

## Beschreibung

Die Erfindung betrifft eine adaptive, externe/separate Arbeitskanaleinrichtung zur wahlweisen Montage am Außenumfang eines Endoskops insbesondere der flexiblen Schaftbauart und ein Endoskop der (flexiblen) Schaftbauart mit einem solchen externen Arbeitskanal (englisch Additional Working Channel, kurz AWC). Der AWC ist dazu vorbereitet, auf/um ein Endoskop/Endoskopkopf montiert zu werden und zu diesem in beliebiger oder vordefinierter Stellung ausgerichtet zu werden.

Ein Endoskop und eine mit dem Endoskop verbundene Schlauchanordnung ist bereits aus der DE 10 2009 014 178 A1 bekannt. Ferner offenbaren die WO 2005/039427 A1, die EP 1 284 120 A1, die DE 103 56 018 A, die JP 4 441 232 B2 und die JP H07 155 283 A jeweils ein Endoskop mit einem daran befestigbaren Arbeitskanal.

Die Erfindung betrifft eine Arbeitskanaleinrichtung mit den Merkmalen des Patentanspruchs 1. Genauer gesagt betrifft die Erfindung eine Arbeitskanaleinrichtung zur wahlweisen Montage am Außenumfang eines Endoskops, insbesondere der flexiblen Schaftbauart, mit einem rohr- oder schlauchförmigen Arbeitskanal, einem am Arbeitskanal fixierten oder fixierbaren distalen Befestigungsmittel, das dafür ausgebildet ist, das Endoskop klemmend oder einspannend zu umgreifen, einem am Arbeitskanal fixierten oder fixierbaren, proximalen Befestigungsmittel, das dafür ausgebildet ist, das Endoskop an seinem Endoskopgriff klemmend oder einspannend zu umgreifen und einem hülsenförmigen Adapter, der am proximalen Endbereich des Arbeitskanals der Arbeitskanaleinrichtung montiert oder montierbar ist und der zusätzlich eine Anschlussstelle/Schnittstelle für das Anschließen medizinischer Geräte bildet, wobei der Adapter das proximale Befestigungsmittel integral als zwei sich von diesem weg erstreckende Flügel oder Laschen oder als zwei bügelförmige Ausleger ausbildet.

Der erfindungsgemäße AWC weist im Wesentlichen vier Komponenten auf.
- Eine erste Komponente ist durch eine distale Endkappe oder Schelle/Federclip realisiert, welche (hinsichtlich ihres inneren Durchmessers) dafür ausgebildet ist, eine Steck-/Schnapp- oder Clipverbindung mit dem Distalende (Endoskopkopf) des (herkömmlichen/konventionellen) Endoskops herzustellen. Neben der Schnappverbindung ist die Endkappe oder Schelle/Federclip vorzugsweise zusätzlich mit einem Klebeband fixierbar, das wahlweise mit der Endkappe oder Schelle/Federclip fest verbunden ist oder als separates Zusatzbauteil beigelegt ist.
- Eine zweite Komponente ist durch einen Schlauch oder eine flexible Rohrhülse realisiert, welche(r) den zusätzlichen Arbeitskanal darstellt und welche(r) mit der Endkappe oder Schelle/Federclip verbindbar oder fest verbunden ist. Der Schlauch/Rohrhülse ist vorzugsweise am distalen Ende zugeschnitten bzw. entsprechend der aktuellen Axiallänge des Endoskopschafts abgelängt bzw. ablängbar. Überdies können an dem Schlauch/Rohrhülse, in vorzugsweise gleichmäßigen Abständen, weitere Angriffspositionen/Montagemittel, beispielsweise für weitere Klebestreifen oder Clipse oder Klettbänder vorhanden/vorgesehen sein, mittels denen der Schlauch am Endoskopschaft zwischen dem Endoskopkopf und seinem standardgemäß vorhandenen Endoskopgriff fixierbar ist.
- Eine dritte Komponente betrifft ein lösbares Befestigungsmittel zur temporären/wahlweisen Montage des proximalen Endabschnitts des separaten Schlauchs/Rohrhülse am Endoskopgriff, beispielsweise bestehend aus zwei klammer- oder flügelartigen Montagelaschen, die am proxiamelen Endabschnitt des Schlauchs/Rohrhülse (nach dessen korrekter Ablängung) montierbar sind und an ihren freien Enden Befestigungen
   (Ösen/Durchgangslöcher/Haken/Knöpfe und dergleichen) aufweisen, an denen vorzugsweise ein Zugband wie Klettband/Gummiband/Gurt, usw. befestigbar ist.
- Eine vierte Komponente ist durch einen Adapter realisiert, welcher eine Schnittstelle für genormte Verbindungssysteme aufweist. Dieser Adapter ist zunächst dafür vorbereitet, hülsenartig auf das proximale Ende des Schlauchs/Rohrhülse aufgesetzt/angesetzt zu werden, wobei der Adapter auch gleichzeitig die zwei klammer- oder flügelartigen Montagelaschen vorzugsweise einstückig trägt. Somit sind auch herkömmliche Verbindungssysteme mit dem Adapter koppelbar, um wirtschaftlich den AWC mit anderen medizinischen Geräten wie Spritzen/Pumpen etc. zu koppeln.

In anderen Worten betrifft die Erfindung ein Nachrüstset zum Ausstatten eines Endoskops mit einem zusätzlichen Arbeitskanal (AWC). Das Set beinhaltet besagten zusätzlichen Arbeitskanal, der als flexibler Schlauch ausgebildet ist und außenumfänglich am Schaft des nachgerüsteten Endoskops angeordnet und befestigt wird; ein distales Befestigungsmittel, welches dazu ausgebildet ist, das distale Ende des zusätzlichen Arbeitskanals mit dem distalen Ende eines nachgerüsteten Endoskops zu verbinden; ein proximales Befestigungsmittel, welches dazu ausgebildet ist, das proximale Ende des zusätzlichen Arbeitskanals am Griffteil eines nachgerüsteten Endoskops festzulegen; und einen Adapter als Schnittstelle für medizinischer Instrumente oder Geräte, bspw. Spritzen.

Gemäß einem Aspekt der Erfindung kann als zusätzliche (fünfte) Komponente ein Überschlauch vorgesehen sein, welcher dazu angepasst und ausgebildet ist, über den Arbeitskanal und das mit diesem adaptierte/nachgerüstete Endoskop gezogen werden zu können. Der Überschlauch umhüllt also das Endoskop und den an diesem angeordneten zusätzlichen Arbeitskanal und kann somit verhindern, dass Gewebe zwischen diesen beiden Komponenten eingeklemmt wird, was zu Komplikationen führen kann (bspw. Zerreißen von Gewebe und Perforation).

Gemäß einer bevorzugten Ausgestaltung der Erfindung kann das distale Befestigungsmittel zumindest einen Endoskopkopf-Anschlag ausbilden, der das distale Befestigungsmittel gegenüber dem Endoskopkopf durch einen Formschluss in dessen Axialrichtung festlegt. Bevorzugt kann dieser Endoskopkopf-Anschlag derart ausgebildet sein, dass er an einer Stirnseite / Strinfläche des Endoskopkopfs eines mit der Arbeitskanaleinrichtung adaptierten Endoskops anschlägt. Auf diese Weise wird ein Formschluss erzeugt, der beim Einführen eines mit dem AWC nachgerüsteten Endoskops ein Verrutschen des zusätzlichen Arbeitskanals in Richtung proximal zuverlässig verhindert.

Gemäß einer weiter bevorzugten Ausführungsform kann das distale Befestigungsmittel eine erste Aufnahme mit zumindest teilkreisförmigem Querschnitt zur Aufnahme des Arbeitskanals und eine zur ersten Aufnahme benachbarte, parallel ausgerichtete, zweite Aufnahme mit zumindest teilkreisförmigem Querschnitt zur Aufnahme des Endoskopkopfes aufweisen.

Gemäß einer bevorzugten Ausgestaltung der Erfindung kann die erste Aufnahme eine zur zweiten Aufnahme hin geöffnete oder geschlitzte Hülsenform haben und die zweite Aufnahme durch zwei sich sichelförmig von der ersten Aufnahme weg erstreckende Klemmarme ausgebildet sein. Das distale Ende des zusätzlichen Arbeitskanals kann entsprechend in die hülsenförmige erste Aufnahme geschoben werden, während die sichelförmigen Klemmarme mit ihren Innenflächen die zweite Aufnahme bilden, welche eine distale Spitze eines nachgerüsteten Endoskops klemmend umgreifen können. Durch seine geschlitzte Hülsenform kann die erste Aufnahme mittels seiner internen Elastizität als Federelement zur Bereitstellung (zumindest eines Teils) der Klemmkraft der Klemmarme dienen.

Gemäß einer Weiterbildung der vorstehenden Ausführungsform können die sichelförmigen Klemmarme des distalen Befestigungsmittels in einer Ebene einer Stirnfläche eines adaptierten Endoskops mit ihren Außenflächen eine Kreisform definieren und die Außenfläche der die erste Aufnahme definierenden Hülse kann in Radialrichtung um nicht mehr als den halben Durchmesser der ersten Aufnahme über diese Kreisform vorragen / hervorstehen. In anderen Worten kann sich der zusätzliche Arbeitskanal in die frontale Silhouette des distalen Befestigungsmittels einfügen. Im Gegensatz zu herkömmlichen AWCs, bei denen das distale Arbeitskanalende mit seinem gesamten Durchmesser vom Außenumfang der distalen Kappe radial absteht, ergeben sich durch die beschriebene konstruktive Gestaltung bessere Gleiteigenschaften beim Einführen eines nachgerüsteten Endoskops.

Gemäß der vorliegenden Erfindung ist das proximale Befestigungsmittel integral mit dem Adapter ausgebildet. Der Adapter weist demnach die zwei klammer-, bügel- oder flügelartigen Montagelaschen oder auch Ausleger (vorzugsweise stoffeinstückig) auf, welche zur ergonomisch günstigen Befestigung des Adapters am Handgriff des Endoskops dienen. Hierbei weisen die Ausleger in einer ersten Ausführungsform Löcher und der Adapter einen Haken oder Knopf auf, welche sämtlich zum Eingriff für eine Klettbandverbindung oder anderweitig gestalteter (Spann-)Gurt dienen. In einer zweiten alternativen Ausführungsform haben die Ausleger Haken (hakenförmige Verlängerungen oder Schnallen), die zum Einschnappen oder Einlegen des Endoskopgriffs in die hakenförmige Verlängerungen oder Schnallen dienen und zusätzlich ein Einhaken eines zusätzlichen elastischen Elements/Gurts/Bandes/Gummis ermöglichen, um den Griff fest einspannen zu können.

Gemäß einer bevorzugten Ausgestaltung der Erfindung können die klammer-, bügel- oder flügelartigen Montagelaschen oder Ausleger an ihren freien Enden voneinander abgewandte Haken ausbilden, welche mit einem Gummiring oder -band gegeneinander verspannt werden können, um die den Endoskopgriff umgreifenden Montagelaschen oder Ausleger zu sichern. Bevorzugt kann hierbei zumindest eine der Montagelaschen oder einer der Ausleger benachbart zu dem an seinem freien Ende angeordneten Haken in seinem Randbereich einen Halteschlitz aufweisen. Dieser Halteschlitz ist zum Rand der Montagelasche oder des Auslegers hin geöffnet, wobei diese Öffnung eine Engstelle (Flaschenhals) ausbildet. Der Gummiring bzw. das Gummiband wird durch besagte Engstelle geschnappt (kann darin vormontiert sein) und folglich in dem Halteschlitz gehalten. Dies verhindert, dass der Gummiring bzw. das Gummiband bei der Montage des AWCs am Endoskopgriff aus den Hakenrutscht und zu Boden fällt (unsteril wird).

Gemäß einem weiteren Aspekt kann der Adapter ein integriertes Ventil aufweisen bzw. kann ein Ventil auf dem Adapter montiert sein, welches dazu dienen soll, den Arbeitskanal abzudichten, wenn ein Instrument im Arbeitskanal verwendet wird und / oder so abzudichten, dass keine Luft/Flüssigkeit aus dem Körper des Patienten entweichen/fließen kann.

Die Erfindung wird nachstehend anhand bevorzugter Ausführungsbeispiele unter Bezugnahme auf die begleitenden Figuren näher erläutert. Die Figuren sind lediglich schematischer Natur und dienen ausschließlich dem Verständnis der Erfindung. Die gleichen Elemente sind mit denselben Bezugszeichen versehen. Es zeigen:

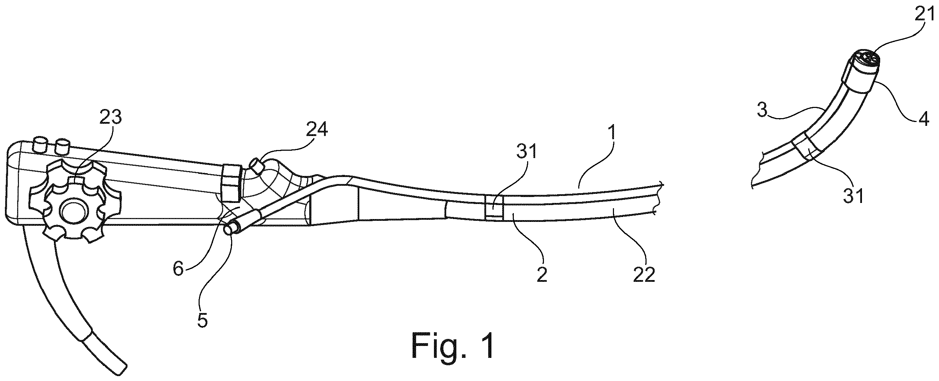

- Fig. 1: ein Endoskop, aufweisend einen Endoskopgriff, einen Endoskopschaft sowie eine Arbeitskanaleinrichtung gemäß einer ersten Ausführungsform der vorliegenden Erfindung;
- Fig. 2: eine distales Befestigungsmittel bzw. eine distale Endkappe erfindungsgemäßen Arbeitskanaleinrichtung gemäß der ersten Ausführungsform;
- Fig. 3: ein rohr- oder schlauchförmiger Arbeitskanal mit der distalen Endkappe gemäß der ersten Ausführungsform in einem an einem Endoskop montierten Zustand;

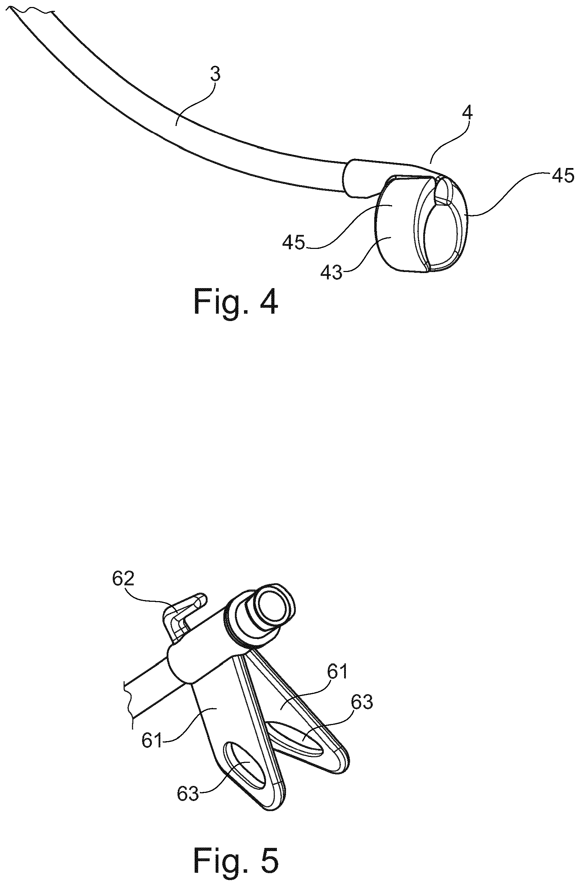

- Fig. 4: der rohr- oder schlauchförmige Arbeitskanal mit der distalen Endkappe gemäß der ersten Ausführungsform ohne Endoskop;
- Fig. 5: ein proximales Befestigungsmittel der Arbeitskanaleinrichtung gemäß der ersten Ausführungsform;

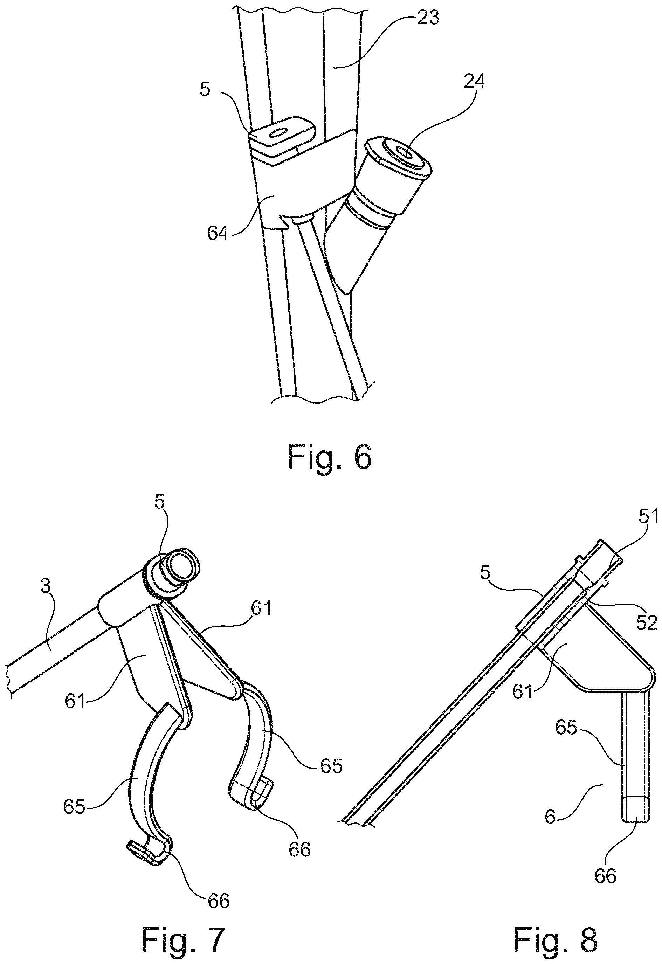

- Fig. 6: das proximale Befestigungsmittel der Arbeitskanaleinrichtung gemäß der ersten Ausführungsform in einem an einem Endoskopgriff befestigten Zustand;
- Fig. 7: ein proximales Befestigungsmittel einer Arbeitskanaleinrichtung gemäß einer zweiten Ausführungsform;
- Fig. 8: eine Schnittansicht des proximalen Befestigungsmittels der Arbeitskanaleinrichtung gemäß der zweiten Ausführungsform;
- Fig. 9: eine Schnittansicht der distalen Endkappe in der bevorzugten Ausführungsform;
- Fig. 10: eine Schnittansicht des proximalen Befestigungsmittels der Arbeitskanaleinrichtung gemäß der ersten Ausführungsform;
- Fig. 11: eine Frontalansicht einer an einem Endoskopkopf montierten distalen Endkappe gemäß einer zweiten Ausführungsform;
- Fig. 12: eine perspektivische Ansicht der Endkappe gemäß der zweiten Ausführungsform;
- Fig. 13: eine perspektivische Ansicht der an einem Endoskopkopf montierten Endkappe gemäß der zweiten Ausführungsform;
- Fig. 14: ein proximales Befestigungsmittel einer Arbeitskanaleinrichtung gemäß einer dritten Ausführungsform;
- Fig. 15: ein Detail eines am proximalen Befestigungsmittel angeordneten Adapters mit Verschluss und Ventil;
- Fig. 16: eine perspektivische Ansicht des proximalen Befestigungsmittels gemäß der dritten Ausführungsform;
- Fig. 17: eine Ansicht einer kompletten Arbeitskanaleinrichtung gemäß einer Ausführungsform ohne Endoskop; und
- Fig. 18: ein Detail einer Arbeitskanaleinrichtung gemäß einer Ausführungsform mit Überschlauch.

Grundsätzlich betrifft die Erfindung eine Arbeitskanaleinrichtung 1 zum Nachrüsten eines (handelsüblichen) Endoskops 2 der flexiblen Schaftbauart mit einem zusätzlichen Arbeitskanal 3, einem sogenannten AWC (Additional Working Channel). Derartige Endoskope 2 sind dem Stand der Technik hinlänglich bekannt und werden deshalb im Folgenden nur oberflächlich beschrieben. So weist ein solches Endoskop 2 in der Regel einen Endoskopkopf 21 auf, in welchem diverse, im Folgenden nicht näher erläuterte, Funktionseinheiten 25, wie z. B. ein Ausgang eines primäreren (endoskopinternen) Arbeitskanals, eine Optik zur Bildgebung und/oder Leuchtmittel, angeordnet sind. Weiter weist ein solches Endoskop 2 zumeist einen flexiblen Schaft 22 mit passiv biegesteifen Abschnitten und vorzugsweise einem aktiv abkrümmbaren Abschnitt (Deflecting) sowie einen Handgriff 23 mit diversen Bedienelementen und Anschlüssen zum Anschließen der diversen Funktionseinheiten 25 an eine Basisstation oder dergleichen auf.

Der zusätzliche Arbeitskanal 3 der erfindungsgemäßen Arbeitskanaleinrichtung 1 wird durch einen Schlauch 3 (bzw. eine rohrförmige Hülse 3) gebildet, der im Gebrauch an den Endoskopschaft 22 angelegt wird und sich entlang und außerhalb dessen vom Handgriff 23 zum Endoskopkopf 21 erstreckt, sodass durch den so gebildeten zusätzlichen externen Arbeitskanal (minimalinvasive) Werkzeuge und/oder Medien von der Anwenderseite aus in einen Patienten hineingeführt werden können.

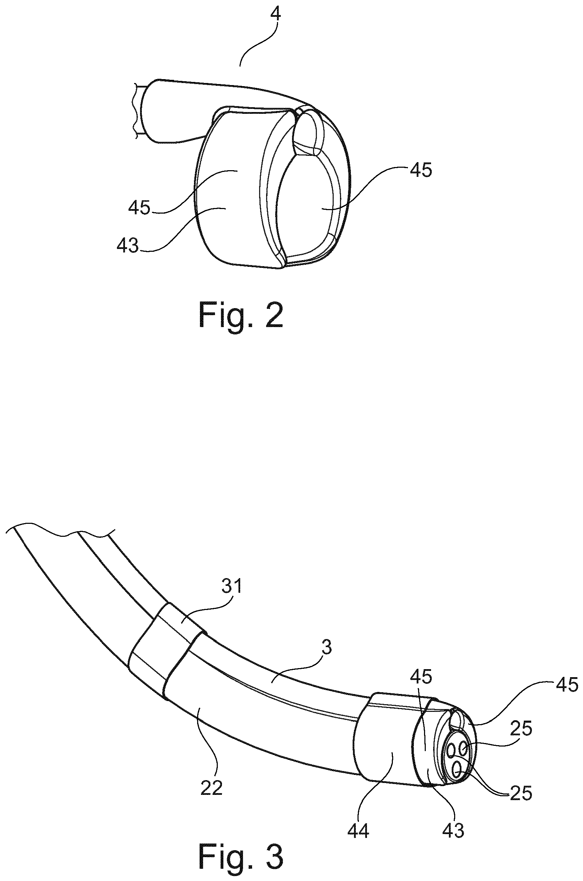

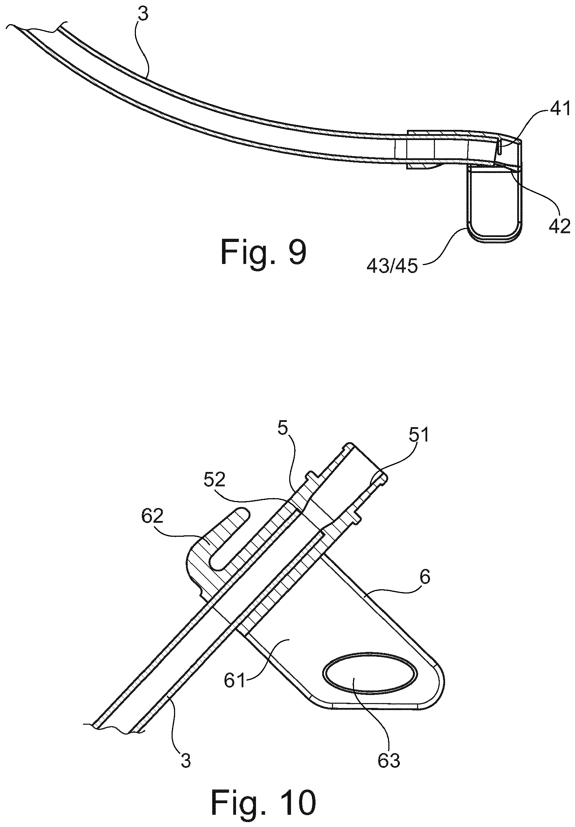

Bei einem bevorzugten Ausführungsbeispiel wird der Arbeitskanal/Schlauch 3 zunächst abgelängt/abgeschnitten und an die Länge des Endoskopschafts 22 angepasst. Zum Festlegen der Ausrichtung und der axialen Lage des distalen Ausgangs des Arbeitskanals/Schlauchs 3, ist am distalen Ende des Arbeitskanals/Schlauchs 3 ein distales Befestigungsmittel (Distalkappe) 4 vorgesehen. Dieses umgreift den Außenumfang des Endoskopkopfs 21 teilkreisförmig, vorzugsweise um etwa 4/5 des Endoskopkopf-Aussenumfangs, mit einem integral ausgebildeten Befestigungsabschnitt 43, der zwei sichelförmige Klemmarme 45 ausbildet und kann so klemmend an diesem festgelegt/festgeclipst werden (Siehe Fig. 3). Wie das Schnittbild (Fig. 9) des distalen Befestigungsmittels 4 zeigt, wird dieses hülsenartig auf den Arbeitskanal/Schlauch 3 aufgeschoben und über einen Anschlag 41 definiert gegenüber diesem festgelegt. Vorzugsweise bildet der Arbeitskanal/Schlauch 3 durch seine interne Elastizität eine Klemmwirkung mit der hülsenartigen Aufnahme des distalen Befestigungsmittels 4 aus. Die hülsenartige Aufnahme für den Arbeitskanal/Schlauch 3 ist an ihrer dem Befestigungsabschnitt 43 zugewandten Seite geschlitzt und dient hierdurch zugleich als Federelement, welches durch seine Eigenelastizität ein Auslenken der sichelförmigen Klemmarme 45 unter Überwindung einer definierten Vorspannkraft ermöglicht. Der teilkreisförmige Befestigungsabschnitt 43 bildet ebenfalls einen Anschlag 42 aus, welcher vorzugsweise um die Teilkreisform des Befestigungsabschnitts 43 umläuft und komplementär zur distalen Endumfangskante des Endoskopkopfs 21 ausgebildet ist, um das distale Befestigungsmittel 4 gegenüber dem Endoskopkopf 21 axial definiert festzulegen. Der Befestigungsabschnitt 43 des distalen Befestigungsmittels 4 wechselwirkt hauptsächlich an der umfangsseitigen Aussenfläche des Endoskopkopfs 21 mit diesem, um die in der Stirnfläche des Endoskopkopfs 21 liegenden Funktionseinheiten freigelegt zu lassen bzw. nicht zu obstruieren. Bevorzugt kann das distale Befestigungsmittel 4 zusätzlich mit einem Tape 44 gegen ein unbeabsichtigtes Lösen durch exzessive Krafteinwirkung gesichert werden.

Der Befestigungsabschnitt 43 bzw. dessen Klemmarme 45 setzen in Fig. 3 in einem oberen, vom adaptierten Endoskop 2 abgewandten Bereich an, sodass die hülsenförmige Aufnahme für den Schlauch 3 in Radialrichtung nicht weit über die Klemmarme 45 vorsteht. Es ergibt sich hierdurch eine vergleichsweise glatte/stromlinienförmige Außenkontur der frontalen Silhouette des distalen Befestigungsmittels 4, insbesondere in Kombination mit einem abgerundeten Design der distalen Umfangskanten der Distalkappe 4.

Um ein Anliegen des Arbeitskanals/Schlauchs 3 am Endoskopschaft 22 sicherzustellen, wird der Arbeitskanal/Schlauch 3 mit, bevorzugt gleichmäßig beabstandeten, Tapes/Klebebändern 31 oder Clipsen (nicht dargestellt) an diesem fixiert.

An der proximalen, dem Anwender zugewandten Öffnung des Arbeitskanals/Schlauchs 3 wird/ist ein vorzugsweise hülsenförmiger Adapter 5 montiert, welcher einen ggf. genormten Anschluss/Schnittstelle 51 für medizinische Geräte bildet. Im dargestellten Ausführungsbeispiel ist dies etwa ein Luer-Konus zum Anschließen von Spritzen und dergleichen. Der hülsenförmige Adapter 5 bildet, an seiner Innenseite umlaufend, ebenfalls einen Anschlag 52 zum axialen Festlegen des Arbeitskanals/Schlauchs 3 gegenüber dem proximalen Adapter 5 aus. Auch hier kann vorteilhafter Weise die Eigenelastizität des Schlauchs 3 einen zusätzlichen Kraftschluss mit dem Adapter 5 ausbilden.

Proximal wird die Arbeitskanaleinrichtung 1 mit einem proximalen Befestigungsmittel 6, vorzugsweise am Endoskopgriff 23, befestigt, insbesondere so, dass der Adapter 5 in etwa auf Höhe des Eingangs 24 des regulären Arbeitskanals des umgerüsteten Endoskops 20 liegt. Das Befestigungsmittel 6 ist erfindungsgemäß in den gezeigten und im Folgenden beschriebenen Ausführungsbeispielen integral mit dem Adapter 5 gefertigt, kann aber auch als separates modulares Bauteil ausgeführt sein.

Eine erste , erfindungsgemäße Ausführungsform des proximalen Befestigungsmittels 6 sieht zwei Flügel/Befestigungslaschen 61 vor, welche sich vom Außenumfang des hülsenförmigen Adapters 5 weg erstrecken, um im montierten Zustand einen Endoskopgriff 23 zumindest teilweise oder vollständig umgreifen zu können. Auf der den Flügeln 61 gegenüberliegenden Seite des Außenumfangs des Adapters ist ein Haken 62 angeordnet, welcher dazu dient, ein Klettband 64 (oder einen anderweitigen Spanngurt) festzulegen. Dieses Klettband/Spanngurt 64 kann anschließend, über den Haken 62 fixiert, durch Ösen 63 an den freien Enden der Flügel 61 geführt werden, um das proximale Haltemittel 6 und damit die Arbeitskanaleinrichtung 1 am Handgriff 23 festzuzurren/zu fixieren.

Eine zweite , erfindungsgemäße Ausführungsform des proximalen Befestigungsmittels 6 sieht zwei insbesondere bügelförmig (konvex gekrümmte), Ausleger 65 an den freien Enden der Flügel 61 vor, welche dazu ausgebildet sind, einen Endoskopgriff 23 spangen- bzw. zangenartig (teilkreisförmig) zu umgreifen. Die Flügel 61 und/oder die Ausleger 65 besitzen eine gewisse Eigenelastizität, sodass der Handgriff 23 zwischen diese eingeschnappt werden kann. Zur sicheren Fixierung der Ausleger 65 sind an deren freien Enden gegenläufig (konkav) gekrümmte Haken 66 vorgesehen. Mit Hilfe dieser Haken 66 können die freien Enden der Ausleger 65 mittels eines Gummirings, eines O-Rings oder dergleichen gegen ein Auseinanderspreizen gesichert werden, wodurch das proximale Befestigungsmittel 6 sicher am Handgriff 23 des Endoskops 20 gehalten wird. In anderen Worten bilden die Ausleger 65 mit den an ihren freien Enden angeordneten Haken 66 im Wesentlichen eine Ω-Form aus, deren Füße mit einem zusätzlichen elastischen Band gegenüber einer öffnenden Bewegung gesichert sind.

Eine dritte Ausführungsform der Arbeitskanaleinrichtung 1 unterscheidet sich zunächst darin, dass die Arbeitskanaleinrichtung einen zusätzlichen Überschlauch 7 aufweist. Weiter ist das Design des distalen Befestigungsmittels (der Distalkappe) 4, des Adapters 5 sowie des proximalen Befestigungsmittels 6 gegenüber der ersten und der zweiten Ausführungsform leicht abgeändert, wie nachstehend genauer erläutert wird.

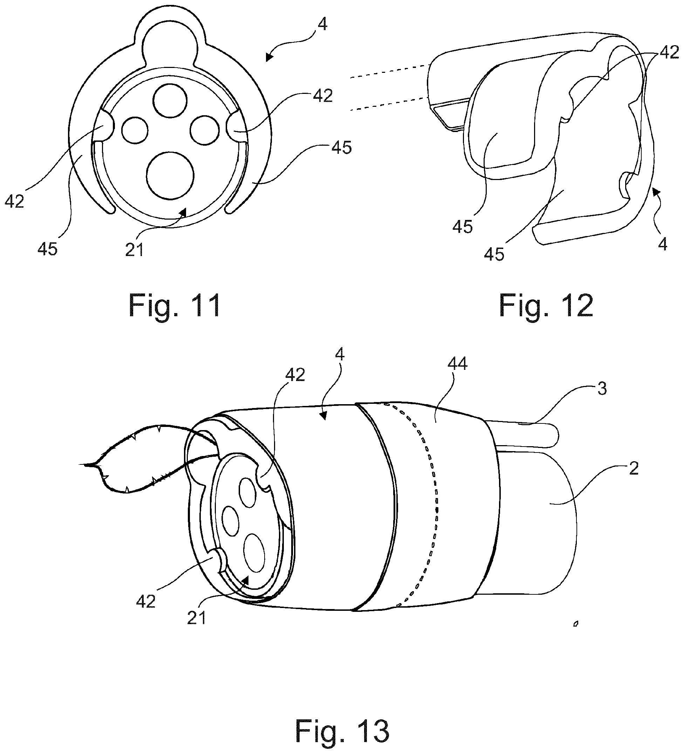

Wie die Figuren 11 bis 13 verdeutlichen, sind an dem distalen Befestigungsmittel bzw. der Distalkappe 4 der dritten Ausführungsform der Erfindung laschenartige Endoskopkopfanschläge 42 vorgesehen, die sich von den sichelförmigen Klemmarmen 45 des Befestigungsabschnitts 43 aus nach radial innen erstrecken und einen Anschlag mit der Stirnfläche des Endoskopkopfs 21 bilden, sodass ein Verrutschen der Distalkappe 4 gen proximal zuverlässig verhindert wird. Zusätzlich erleichtern die Endoskopkopfanschläge 42 bei der Montage der Distalkappe 4 an dem Endoskopkopf 21 eine definierte Lage des distalen Ausgangs des zusätzlichen Arbeitskanals 3 bezüglich des Endoskopkopfs 21 herzustellen.

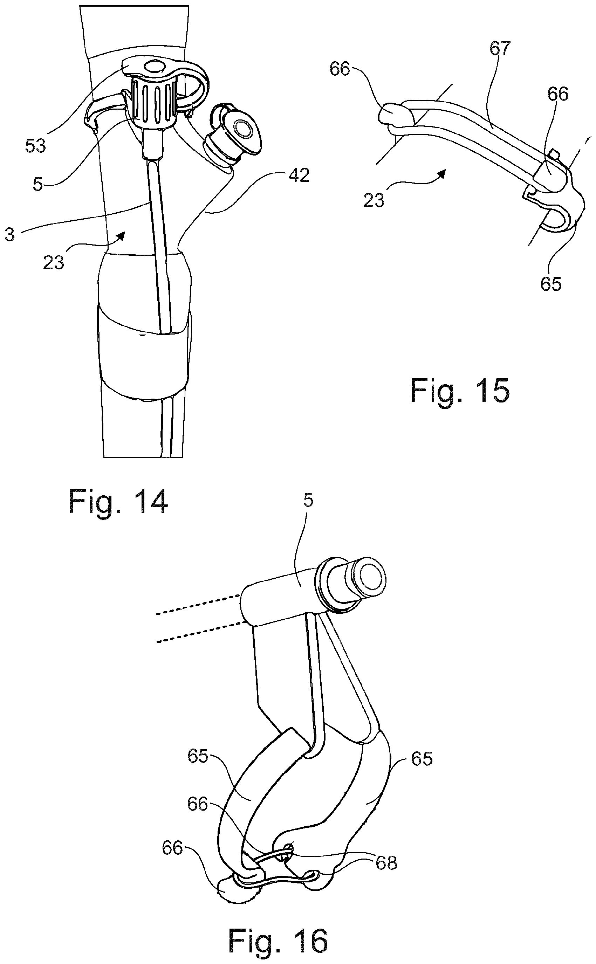

Der in Fig. 14 gezeigte Adapter 5 der Arbeitskanaleinrichtung 1 der dritten Ausführungsform ist zum einen mit einer (integral gefertigten) Kappe 53 verschließbar und weist zum anderen in seinem Inneren eine nicht näher dargestellte Ventileinrichtung auf, die ein ungewolltes Eindringen Fremdkörpern in den Arbeitskanal 3 ebenso verhindert wie ein ungewolltes Austreten von Körperflüssigkeit und dergleichen.

Das in den Figuren 15 und 16 dargestellte proximale Befestigungsmittel 6 der dritten Ausführungsform ähnelt dem der zweiten Ausführungsform, indem es ebenfalls zwei integral mit dem Adapter 5 ausgebildete sichelartige Spangen 65 aufweist, die dazu ausgebildet sind, einen Endoskopgriff 23 zu umgreifen und mittels an ihren distalen Endabschnitten angeformten Haken 66 und eines Gummirings 67 gegeneinander verspannt zu werden. Das proximale Befestigungsmittel 6 der dritten Ausführungsform der Erfindung weist zusätzliche Halteschlitze oder -ösen 68 auf, die benachart zu einem der Haken 66 in den Rand des freien Endes der den Haken 66 tragenden Spange 65 eingeformt sind. Die Halteschlitze 68 sind im Bereich ihrer Öffnung verengt und weiter innenbauchig ausgeführt, sodass der Gummiring 67 nur unter elastischer Verformung durch die Engstelle geschoben/geschnappt werden kann sich im bauchigen Bereich der Halteschlutze 68 jedoch wieder aufweitet. Auf diese Weise wird der Gummiring 67 sicher am proximalen Befestigungsmittel 6 gehalten und kann während der Montage nicht von den Haken 66 rutschen und auf den Boden fallen.

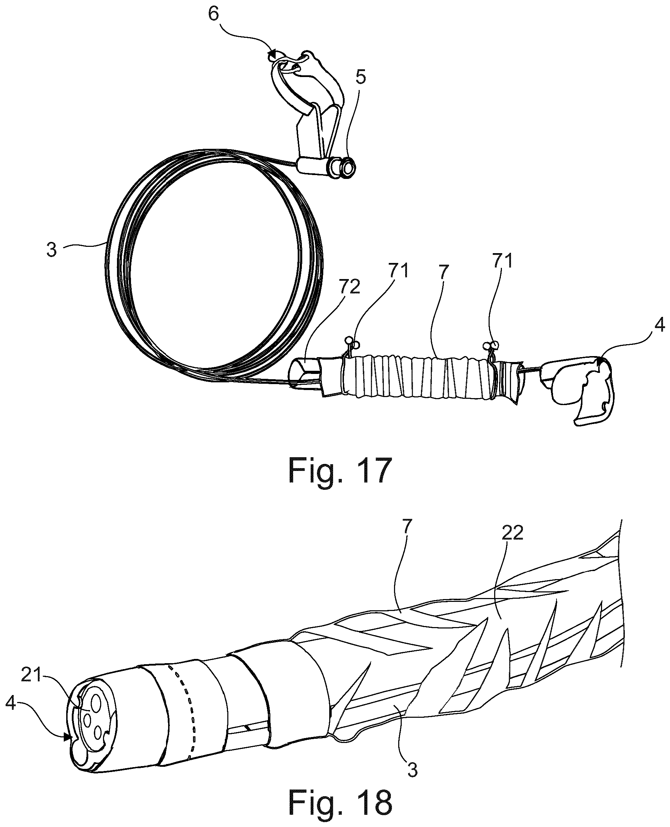

Der bei der dritten Ausführungsform als weitere Komponente hinzugekommene Überschlauch 7 ist am besten in den Figuren 17 und 18 zu erkennen. Wie in Fig. 18 dargestellt ist, wird der Überschlauch 7 proximal hinter dem Endoskopkopf 21 befestigt und zum Endoskopgriff 23 hin über den Endoskopschaft 22 des adaptierten Endoskops 2 und den zusätzlichen Arbeitskanal 3 gezogen. Wie in der Fig. 18 zu erkennen ist, kann der zusätzliche Arbeitskanal 3 bei einem entsprechenden Abkrümmen des Endoskopschafts 22 unter Umständen von diesem etwas abstehen, sodass sich ein Spalt bildet. In einem solchen Fall verhindert der Überschlauch 7, dass Gewebe in besagtem Spalt eingeklemmt wird, was zu einem Gewebetraume führen könnte. Der in Fig. 17 dargestellten Arbeitskanaleinrichtung 1 liegen Kabeldriller 71 zur vorläufigen Fixierung des Überschlauchs 7 während der Montage sowie ein (Führungs-)Röhrchen 72, welches das Aufschieben des gerafften Überschlauchs 7 auf ein zu adaptierendes Endoskop 2 erleichtert, bei.

Im Folgenden wird anhand der vorstehend beschriebenen dritten Ausführungsform ein typischer Montagevorgang der Arbeitskanaleinrichtung 1 an einem Endoskop 2 beschrieben.

Zunächst wird das Endoskop durch das der Arbeitskanaleinrichtung 1 beiliegende Röhrchen 72 des Überschlauchs 7 geschoben.

Anschließend wird das distale Befestigungsmittel (4) auf die Endoskopspitze 21 aufgesetzt, sodass die Anschläge 42 an der Stirnseite der Endoskopspitze 21 anschlagen. Der zu diesem Zeitpunkt noch relativdrehbare Arbeitskanal 3 wird in Umfangsrichtung entsprechend der gewünschten Anwendung positioniert. Hernach wird das distale Befestigungsmittel mit im Set beiliegenden Klebestreifen 31 am Endoskop fixiert.

Der AWC Adapter 5 wird mit dem proximalen Befestigungsmittel 6 rechts oder links (je nach Präferenz) neben dem Endoskop Arbeitskanaleingang am Endoskopgriff 23 befestigt, indem die Ausleger bzw. Spangen 65 oberhalb des Arbeitskanals um den Handgriff gelegt werden. Der Adapter 5 wird dann durch Befestigen des Gummirings 67 an den vorgesehenen Haken 66 an den Spangen 65 gesichert.

Ein weiterer Klebestreifen 31 am Endoskopschaft, ca. 10 cm unterhalb des AWC Adapters 5 befestigt diesen zusätzlich am Endoskopgriff 23

Der distale Kabeldriller 71 wird entfernt, das Schlauchende wird mit einem Klebestreifen 31 auf dem Endoskop 2 mit Abstand zu den Klebestreifen 31 des distalen Befestigungsmittels befestigt und der Überschlauch 7 (Endoskopüberzug) wird mit Hilfe des Röhrchens 72 bis zum proximalen Ende des Endoskops 2 zurückgezogen. Er rollt sich dabei ab. Der Überschlauch 7 wird zum proximalen Ende hin geglättet, so dass am distalen Ende kein überschüssiges Material vorhanden ist. Der verbliebene Kabeldriller 71 und das Röhrchen 72 werden entfernt. Um die Einführung des Systems zu erleichtern, kann die Oberfläche des Überschlauchs 7 reduziert werden, indem die zusätzlich vorhandenen Klebestreifen 31 mit Abstand verteilt auf dem Überschlauch 7 angebracht werden.

### Bezugszeichen

- 1: Arbeitskanaleinrichtung;
- 2: Endoskop;
21 Endoskopkopf / Endoskopspitze;
22 Endoskopschaft;
23 Endoskopgriff;
24 Eingang/Zugang des regulären Arbeitskanals;
25 Funktionseinheiten (Optik, Leuchtmittel, Arbeitskanal etc.);
- 3: Arbeitskanal bzw. Arbeitskanal/Schlauch/flexible Rohrhülse;
31 Befestigungstapes;
- 4: distales Befestigungsmittel/distale Endkappe;
41 distaler Schlauch-Anschlag;
42 Endoskopkopfanschlag;
43 Befestigungsabschnitt;
44 Befestigungstape;
45 Klemmarme
- 5: Adapter;
51 Schnittstelle/Luer-Kegel;
52 proximaler Schlauch-Anschlag;
53 Kappe;
- 6: proximales Befestigungsmittel;
61 Flügel/Befestigungslaschen;
62 Haken;
63 Ösen;
64 (Klett)Gurt;
65 Ausleger / Spangen;
66 Haken;
67 Gummiring;
68 Halteschlitz;
- 7: Überschlauch;
71 Kabeldriller;
72 Röhrchen.

## Patentansprüche

1. Arbeitskanaleinrichtung (1) zur wahlweisen Montage am Außenumfang eines Endoskops (2), insbesondere der flexiblen Schaftbauart, mit
- einem rohr- oder schlauchförmigen Arbeitskanal (3),
- einem am Arbeitskanal (3) fixierten oder fixierbaren distalen Befestigungsmittel (4), das dafür ausgebildet ist, das Endoskop (2) klemmend oder einspannend zu umgreifen,
- einem am Arbeitskanal (3) fixierten oder fixierbaren, proximalen Befestigungsmittel (6), das dafür ausgebildet ist, das Endoskop (2) an seinem Endoskopgriff (23) klemmend oder einspannend zu umgreifen und
- einem hülsenförmigen Adapter (5), der am proximalen Endbereich des Arbeitskanals (3) der Arbeitskanaleinrichtung (1) montiert oder montierbar ist und der zusätzlich eine Anschlussstelle/Schnittstelle (51) für das Anschließen medizinischer Geräte bildet, **dadurch gekennzeichnet, dass**
der Adapter (5) das proximale Befestigungsmittel (6) integral entweder als zwei sich von diesem weg erstreckende Flügel oder Laschen (61) zum Umgreifen des Endoskopgriffs (23), die jeweils eine Öse (63) zum Führen eines Gurtes oder Bandes (64) ausbilden, oder als zwei bügelförmige Ausleger (65), die dazu ausgebildet sind, den Endoskopgriff (23) klemmend zwischen sich aufzunehmen, ausbildet.

2. Arbeitskanaleinrichtung (1) gemäß Anspruch 1, **dadurch gekennzeichnet, dass** das distale Befestigungsmittel (4) zumindest einen Endoskopkopf-Anschlag (42) ausbildet, der das distale Befestigungsmittel (4) durch einen Formschluss in Axialrichtung gegenüber dem Endoskopkopf (21) festlegt.

3. Arbeitskanaleinrichtung (1) gemäß Anspruch 2, **dadurch gekennzeichnet, dass** der Endoskopkopf-Anschlag (42) derart ausgebildet ist, dass er an einer Stirnseite des Endoskopkopfs (21) eines mit der Arbeitskanaleinrichtung (1) adaptierten Endoskops (2) anschlägt.

4. Arbeitskanaleinrichtung (1) gemäß einem der Ansprüche 1 bis 3, **dadurch gekennzeichnet, dass** das distale Befestigungsmittel (4) eine erste Aufnahme mit zumindest teilkreisförmigem Querschnitt zur Aufnahme des Arbeitskanals (3) und eine zur ersten Aufnahme benachbarte, parallel ausgerichtete, zweite Aufnahme mit zumindest teilkreisförmigem Querschnitt zur Aufnahme des Endoskopkopfes (21) aufweist.

5. Arbeitskanaleinrichtung (1) gemäß Anspruch 4, **dadurch gekennzeichnet, dass** die erste Aufnahme eine zur zweiten Aufnahme hin geöffnete oder geschlitzte Hülsenform hat und die zweite Aufnahme durch zwei sich sichelförmig von der ersten Aufnahme weg erstreckende Klemmarme ausgebildet ist.

6. Arbeitskanaleinrichtung (1) gemäß Anspruch 5, **dadurch gekennzeichnet, dass** die sichelförmigen Klemmarme des distalen Befestigungsmittels (4) in einer Ebene einer Stirnfläche eines adaptierten Endoskops mit ihren Außenflächen eine Kreisform definieren und die Außenfläche der die erste Aufnahme definierenden Hülse in Radialrichtung um nicht mehr als den halben Durchmesser der ersten Aufnahme über diese Kreisform vorragt.

7. Arbeitskanaleinrichtung (1) gemäß einem der Ansprüche 1 bis 6, **gekennzeichnet durch** einen auf der von den Flügeln oder Laschen (61) abgewandten Seite des hülsenförmigen Adapters (5) angeordneten Haken (62) zum Führen eines Gurtes oder Bandes (64).

8. Arbeitskanaleinrichtung (1) gemäß einem der Ansprüche 1 bis 7, **dadurch gekennzeichnet, dass** zumindest einer der bügelförmigen Ausleger (65) an seinem freien Ende einen entgegengesetzt zur Krümmungsrichtung des Auslegers (65) gekrümmten Haken (66) zum Halten eines Gurtes oder Bandes (64) aufweist.

9. Arbeitskanaleinrichtung (1) gemäß einem der Ansprüche 1 bis 8, **dadurch gekennzeichnet, dass** zumindest einer der bügelförmigen Ausleger (65) zumindest einen Halteschlitz in seinem Randbereich aufweist, welcher im Bereich seiner Schlitzöffnung verengt ist, um einen Gurt oder ein Band (64) sicher zu halten.

10. Arbeitskanaleinrichtung gemäß einem der Ansprüche 1 bis 9, wobei das proximale Befestigungsmittel durch vorgenannte bügelförmige Ausleger ausgebildet ist, **dadurch gekennzeichnet, dass** der Adapter (5) ein integriertes Ventil aufweist, welches ein Eintreten von Fremdkörpern in das proximale Ende des Arbeitskanals (3) und / oder ein Austreten von Luft oder Flüssigkeit aus dem proximalen Ende des Arbeitskanals (3) verhindert.

11. Arbeitskanaleinrichtung gemäß einem der Ansprüche 1 bis 10, 2. wobei das proximale Befestigungsmittel durch vorgenannte bügelförmige Ausleger ausgebildet ist, **gekennzeichnet durch** einen zusätzlichen Überschlauch (7), welcher dazu angepasst und ausgebildet ist, über den Arbeitskanal (3) und das mit diesem adaptierte Endoskop (2) gezogen werden zu können.

12. Endoskopsystem mit einem Endoskop (2) mit einem vorzugsweise flexiblen Endoskopschaft (22), an dessen distalem Ende ein Endoskopkopf (21) und an dessen proximalem Ende ein Endoskopgriff (23) angeordnet ist, **gekennzeichnet durch** eine Arbeitskanaleinrichtung (1) mit den Merkmalen eines der Ansprüche 1 bis 11.

## Claims

1. A working channel device (1) for optional mounting on the outer circumference of an endoscope (2), in particular of the flexible shaft design, with
- a tube-shaped or hose-shaped working channel (3),
- a distal fixing means (4) fixed or fixable to the working channel (3) which is configured to grip around the endoscope (2) in a clamping or clasping manner,
- a proximal fixing means (6) fixed or fixable to the working channel (3) and configured to grip around the endoscope (2) on its endoscope grip (23) in a clamping or clasping manner, and
- a sleeve-shaped adapter (5) which is mounted or mountable on the proximal end region of the working channel (3) of the working channel device (1) and which additionally forms a connection point/interface (51) for connecting medical devices, **characterized in that**
the adapter (5) integrally forms the proximal fixing means (6) either as two wings or lugs (61) extending away from it for gripping around the endoscope grip (23), which in turn, each form an eyelet (63) for guiding a tape or strap (64), or forms them as two bow-shaped cantilevers (65) which are configured to accommodate the endoscope grip (23) in a clamping manner between them.

2. The working channel device (1) according to claim 1, **characterized in that** the distal fixing means (4) forms at least one endoscope-head stop (42) which defines the distal fixing means (4) in a form-fit manner in the axial direction with respect to the endoscope head (21).

3. The working channel device (1) according to claim 2, **characterized in that** the endoscope-head stop (42) is configured such that it abuts against a front side of the endoscope head (21) of an endoscope (2) adapted with the working channel device (1).

4. The working channel device (1) according to one of claims 1 to 3, **characterized in that** the distal fixing means (4) has a first accommodation with at least partial circular cross-section for accommodation of the working channel (3) and a second accommodation with at least partial circular cross-section being adjacent to the first accommodation and aligned in parallel for accommodation of the endoscope head (21).

5. The working channel device (1) according to claim 4, **characterized in that** the first accommodation has a sleeve shape open or slotted towards the second accommodation and the second accommodation is formed by two crescent-shaped clamping arms extending away from the first accommodation.

6. The working channel device (1) according to claim 5, **characterized in that** the crescent-shaped clamping arms of the distal fixing means (4) define a circular shape with their outer surfaces in a plane of a front surface of an adapted endoscope, and the outer surface of the sleeve defining the first accommodation protrudes beyond this circular shape in the radial direction by no more than half the diameter of the first accommodation.

7. The working channel device (1) according to one of the claims 1 to 6, **characterized by** a hook (62) arranged on the side of the sleeve-shaped adapter (5) remote from the wings or lugs (61) for guiding a tape or strap (64).

8. The working channel device (1) according to one of the claims 1 to 7, **characterized in that** at least one of the bow-shaped cantilevers (65) has, at its free end, a hook (66), curved in the opposite direction to the direction of curvature of the cantilever (65), for holding a tape or strap (64).

9. The working channel device (1) according to one of the claims 1 to 8, **characterized in that** at least one of the bow-shaped cantilevers (65) has at least one retaining slit in its edge area, which is narrowed in the area of its slot opening in order to securely hold a tape or strap (64).

10. The working channel device according to one of the claims 1 to 9, wherein the proximal fixing means is formed by the aforementioned bow-shaped cantilevers, **characterized in that** the adapter (5) has an integrated valve which prevents foreign bodies from entering the proximal end of the working channel (3) and/or air or liquid from leaking from the proximal end of the working channel (3).

11. The working channel device according to one of the claims 1 to 10, wherein the proximal fixing means is formed by the aforementioned bow-shaped cantilevers, **characterized by** an additional cover hose (7) adapted and configured to be pulled over the working channel (3) and the endoscope (2) adapted therewith.

12. An endoscope system with an endoscope (2) with a preferably flexible endoscope shaft (22), at the distal end of which an endoscope head (21) is arranged and at the proximal end of which an endoscope grip (23) is arranged, **characterized by** a working channel device (1) with the features of one of the claims 1 to 11.

## Revendications

1. Appareil de canal de travail (1) pour le montage au choix au niveau de la périphérie extérieure d'un endoscope (2), en particulier du type à tige souple, avec
- un canal de travail (3) en forme de tube ou de tuyau,
- un moyen de fixation (4) distal fixé ou pouvant être fixé au canal de travail (3) qui est conçu afin d'entourer de manière à bloquer ou enserrer l'endoscope (2),
- un moyen de fixation (6) proximal, fixé ou pouvant être fixé au canal de travail (3) qui est conçu afin d'entourer de manière à bloquer ou enserrer l'endoscope (2) au niveau de sa poignée d'endoscope (23) et
- un adaptateur en forme de douille (5) qui est ou peut être monté au niveau de la zone d'extrémité proximale du canal de travail (3) de l'appareil de canal de travail (1) et qui forme en outre un point de raccordement/une interface (51) pour le raccordement d'appareils médicaux, **caractérisé en ce que**
l'adaptateur (5) forme le moyen de fixation (6) proximal d'un seul tenant soit comme deux ailes ou languettes (61) s'étendant depuis celui-ci pour entourer la poignée d'endoscope (23), ailes ou languettes qui forment respectivement un oeillet (63) pour le guidage d'une sangle ou bande (64), soit comme deux bras (65) en forme d'étrier qui sont conçus afin de recevoir entre eux la poignée d'endoscope (23) de manière à la bloquer.

2. Appareil de canal de travail (1) selon la revendication 1, **caractérisé en ce que** le moyen de fixation (4) distal forme au moins une butée de tête d'endoscope (42) qui fixe le moyen de fixation (4) distal par une liaison par complémentarité de formes dans le sens axial par rapport à la tête d'endoscope (21).

3. Appareil de canal de travail (1) selon la revendication 2, **caractérisé en ce que** la butée de tête d'endoscope (42) est formée de telle manière qu'elle bute contre un côté frontal de la tête d'endoscope (21) d'un endoscope (2) adapté à l'appareil de canal de travail (1).

4. Appareil de canal de travail (1) selon l'une quelconque des revendications 1 à 3, **caractérisé en ce que** le moyen de fixation (4) distal présente un premier logement avec une section transversale au moins en forme de cercle partiel pour le logement du canal de travail (3) et un second logement orienté parallèlement, contigu au premier logement avec une section transversale au moins en forme de cercle partiel pour le logement de la tête d'endoscope (21).

5. Appareil de canal de travail (1) selon la revendication 4, **caractérisé en ce que** le premier logement présente une forme de douille ouverte ou fendue vers le second logement et le second logement est formé par deux bras de serrage s'étendant en forme de faucille depuis le premier logement.

6. Appareil de canal de travail (1) selon la revendication 5, **caractérisé en ce que** les bras de serrage en forme de faucille du moyen de fixation (4) distal définissent une forme circulaire avec leurs surfaces extérieures dans un plan d'une surface frontale d'un endoscope adapté et la surface extérieure de la douille définissant le premier logement fait saillie de cette forme circulaire dans le sens radial de pas plus du demi-diamètre du premier logement.

7. Appareil de canal de travail (1) selon l'une quelconque des revendications 1 à 6, **caractérisé par** un crochet (62) agencé sur le côté éloigné des ailes ou languettes (61) de l'adaptateur (5) en forme de douille pour le guidage d'une sangle ou bande (64).

8. Appareil de canal de travail (1) selon l'une quelconque des revendications 1 à 7, **caractérisé en ce qu'**au moins un des bras (65) en forme d'étrier présente au niveau de son extrémité libre un crochet (66) courbé à l'opposé du sens de courbure du bras (65) pour le maintien d'une sangle ou bande (64).

9. Appareil de canal de travail (1) selon l'une quelconque des revendications 1 à 8, **caractérisé en ce qu'**au moins un des bras (65) en forme d'étrier présente au moins une fente de retenue dans sa zone de bord qui est rétrécie au niveau de son ouverture fendue afin de maintenir en sécurité une sangle ou une bande (64).

10. Appareil de canal de travail selon l'une quelconque des revendications 1 à 9, dans lequel le moyen de fixation proximal est formé par des bras en forme d'étrier précités, **caractérisé en ce que** l'adaptateur (5) présente une soupape intégrée qui empêche une pénétration de corps étrangers dans l'extrémité proximale du canal de travail (3) et/ou une évacuation d'air ou de liquide de l'extrémité proximale du canal de travail (3).

11. Appareil de canal de travail selon l'une quelconque des revendications 1 à 10, dans lequel le moyen de fixation proximal est formé par des bras en forme d'étrier précités, **caractérisé par** une gaine (7) supplémentaire qui est adaptée et formée afin de pouvoir être tirée par le biais du canal de travail (3) et de l'endoscope (2) adapté à celui-ci.

12. Système d'endoscope avec un endoscope (2) avec une tige d'endoscope (22) de préférence souple, à l'extrémité distale de laquelle est agencée une tête d'endoscope (21) et à l'extrémité proximale de laquelle est agencée une poignée d'endoscope (23), **caractérisé par** un appareil de canal de travail (1) avec les caractéristiques selon l'une quelconque des revendications 1 à 11.
